(19) Europäisches Patentamt
European Patent Office
Office européen des brevets

(11) **EP 2 704 590 B1**

(12) **EUROPEAN PATENT SPECIFICATION**

(45) Date of publication and mention of the grant of the patent:
**03.06.2020 Bulletin 2020/23**

(51) Int Cl.:
***A23K 50/40*** (2016.01)  ***A23K 20/163*** (2016.01)
***A23K 20/111*** (2016.01)

(21) Application number: **12721083.9**

(22) Date of filing: **01.05.2012**

(86) International application number:
**PCT/US2012/035921**

(87) International publication number:
**WO 2012/151168 (08.11.2012 Gazette 2012/45)**

(54) **COMPOSITIONS COMPRISING A GLUCOSE ANTI-METABOLITE, BHA, AND/OR BHT**

ZUSAMMENSETZUNGEN MIT EINEM GLUCOSE-ANTI-METABOLIT, BHA UND/ODER BHT

COMPOSITIONS COMPORTANT UN ANTI-MÉTABOLITE DU GLUCOSE, BHA ET/OU BHT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.05.2011 US 201113098756**

(43) Date of publication of application:
**12.03.2014 Bulletin 2014/11**

(73) Proprietor: **IAMS Europe B.V.**
**7742 PB Coevorden (NL)**

(72) Inventors:
• **LUHADIYA, Ashok, Premchand**
**Cincinnati**
**Ohio 45248 (US)**
• **DAVENPORT, Gary, Mictchell**
**Dayton**
**Ohio 45415 (US)**
• **ZHANG, Jin**
**Clayton**
**Ohio 45315 (US)**
• **ROTH, George, S.**
**Pylesville**
**Maryland 21132 (US)**
• **INGRAM, Donald, Keith**
**Baton Rouge**
**Louisiana 70809 (US)**

(74) Representative: **Zimmermann & Partner**
**Patentanwälte mbB**
**Postfach 330 920**
**80069 München (DE)**

(56) References cited:
EP-A1- 2 218 337      CA-A- 1 156 144
FR-A1- 2 663 198      US-A1- 2005 249 841
US-A1- 2009 252 834   US-A1- 2010 233 756

• **GRAJALES-LAGUNES, A., ET AL.: "Stability and Sensory Quality of Srpay Dried Avocado Paste", DRYING TECHNOLOGY., vol. 17, no. 1&2, 1999, pages 317-326, XP002678732, TAYLOR & FRANCIS, PHILADELPHIA, PA. ISSN: 0737-3937**
• **VIKTORA J K ET AL: "Effect of ingested mannoheptulose in animals and man", METABOLISM, CLINICAL AND EXPERIMENTAL, W.B. SAUNDERS CO., PHILADELPHIA, PA, US, vol. 18, no. 2, 1 February 1969 (1969-02-01), pages 87-102, XP026313665, ISSN: 0026-0495, DOI: 10.1016/0026-0495(69)90101-2 [retrieved on 1969-02-01]**

## Description

FIELD

Embodiments of the invention relate to compositions comprising a glucose anti-metabolite, butylated hydroxyanisole (BHA), and/or butylated hydroxytoluene (BHT). More particularly, embodiments of the invention relate to compositions comprising a glucose anti-metabolite, BHA, and/or BHT for companion animals.
The scope of protection is defined by the claims.

BACKGROUND

Biological theories have correctly predicted the finding that a restriction of caloric intake by food deprivation slows down certain undesirable cellular processes in laboratory animals, many associated with aging and age-related diseases.
In particular, caloric restriction has been shown to consistently extend the life span, delay onset and slow tumor progression, and retard physiologic aging in many systems. Indeed, research spanning more than seventy years has shown that caloric restriction is a nutritional intervention that consistently extends longevity in animals. See Weindruch and Walford, "The Retardation of Aging and Disease by Dietary Restriction," Springfield, IL: Charles C. Thomas (1988); Yu, "Modulation of Aging Processes by Dietary Restriction," Boca Raton: CRC Press (1994); and Fishbein, "Biological Effects of Dietary Restriction," Springer, New York (1991). These effects of caloric restriction on life span and tumorigenesis have been reported numerous times since the early studies of McKay. See McKay et al., "The Effect of Retarded Growth Upon the Length of Lifespan and Upon Ultimate Body Size," J. Nutr., Vol. 10, pp. 63 - 79 (1935). Indeed, over the past two decades, a resurgence of interest in caloric restriction in gerontology has led to the general acceptance that this dietary manipulation slows physiologic aging in many systems. See Weindruch and Walford, "The Retardation of Aging and Disease by Dietary Restriction," Springfield, IL: Charles C. Thomas (1988); Yu, "Modulation of Aging Processes by Dietary Restriction," Boca Raton: CRC Press (1994); and Fishbein, "Biological Effects of Dietary Restriction," Springer, New York (1991) and Masoro, E.J. "Overview of Caloric Restriction and Ageing," Mech. Aging Dev., Vol. 126, pp 913-922 (2005).
Reductions in fasting glucose and insulin levels and improvements in insulin sensitivity are readily measured biomarkers of caloric restriction. Calorically restricted rodents exhibit lower fasting glucose and insulin levels, and the peak glucose and insulin levels reached during a glucose challenge are reduced in those on caloric restriction. See Kalant et al., "Effect of Diet Restriction on Glucose Metabolism and Insulin Responsiveness and Aging Rats," Mech. Aging Dev., Vol. 46, pp. 89 - 104 (1988). It is also known that hyperinsulinemia is a risk factor associated with several such disease processes, including heart disease and diabetes (Balkau and Eschwege, Diabetes Obes. Metab. 1 (Suppl. 1): S23 - 31, 1999). Reduced insulin levels and body temperature are two of the most reliable indicators of this altered metabolic profile (Masoro et al., J. Gerontol. Biol. Sci. 47:B202-B208, 1992); Koizumi et al., J. Nutr. 117: 361 - 367, 1987; Lane et al., Proc. Nat. Acad. Sci. 93:4154 - 4164, 1996).
Glucose anti-metabolites such as 2-deoxy- D-glucose are compounds related to glucose. However, due to structural differences from glucose such compounds block or inhibit certain aspects of carbohydrate metabolism and may therefore mimic the effects of caloric restriction (Rezek et al., J. Nutr. 106:143 - 157, 1972). These anti-metabolites exert a number of physiological effects, including reduction of body weight, decrease in plasma insulin levels, reduction of body temperature, retardation of tumor formation and growth, and elevation of circulating glucocorticoid hormone concentrations. (For a review see Roth et al., Ann. NY Acad. Sci. 928:305 - 315, 2001). These physiological effects result from inhibition of carbohydrate metabolism.
Butylated hydroxytoluene (BHT) and butylated hydroxyanisole (BHA) are lipophilic organic compounds, containing conjugated aromatic rings, which largely confer their respective antioxidant properties, and in fact the former may be considered a synthetic analog of Vitamin E. The latter is actually a mixture of two isomeric organic compounds. 2-tert-butyl-4-hydroxyanisole and 3-tert-butyl-4-hydroxyanisole. Neither BHT nor BHA is naturally occurring. BHT is prepared either by the reaction of *p*-cresol (4-methylphenol) with isobutylene (2-methylpropene) or from 2,6-di-*tert*-butylphenol by hydroxymethylation or aminomethylation followed by hydrogenolysis, and BHA is prepared from 4-methoxyphenol and isobutylene.
Both BHT and BHA are used as preservatives in foods, medicines, cosmetics, and related products because of their antioxidative actions. FR 2 663 198 relates to the use of antioxidant compounds chosen from BHA, BHT and ascorbic acid for increasing the resistance of fruits and vegetables to fungal infections, in particular to Colletotrichum gloeosporioides, Diplodia natalensis and Alternaria tenuis. As above, their conjugated aromatic rings stabilize and sequester free radicals, thereby preventing or suppressing autoxidation of unsaturated organic materials. Based on some biological endpoints, BHT and BHA may act both similarly (antioxidants) and synergistically (BHA protection against membrane damage leading to elevated insulin levels, and BHT protection against membrane damage impairing insulin action). Thus, BHA and BHT may likely be considered interchangeable, separately, or in combination for the applications suggested below. Their "primary" effects may, therefore, be considered to be oxygen free radical scavenging, and subsequent biological endpoints as "secondary" results of this oxidative protection. Grajales-Lagunes et al., Drying Technology 17(1 &2), 317-326 (1999) relates to the stability and sensory quality of spray dried avocado paste. The Avocado paste was spray dried and BHA and BHT applications were tested for obtaining stable avocado powders stored at ambient temperatures.

Without being limited by theory, it is thought that a number of potentially synergistic effects and benefits may result if BHT and/or BHA are included in glucose anti-metabolite calorie restriction mimetic formulations and compositions. For example, it is thought that BHT and BHA both can act synergistically with a glucose anti-metabolite to potentiate its effects on insulin sensitivity, insulin signaling, circulating insulin levels, and the maintenance of muscle mass and muscle functionality. Moreover, it has been reported that low dosage of BHA and BHT may be protective for cancer and inhibit carcinogenesis. See Botterweck et al., "Intake of Butylated Hydroxyanisole and Butylated Hydroxytoluene and Stomach Cancer Risk: Results from Analyses in the Netherlands Cohort Study," Food and Chemical Toxicology, 38 (2000) 599-605. It is thus possible that using such a combination may reduce the required dosages of each antioxidant compounds and the glucose anti-metabolite to provide expected benefits since glucose anti-metabolites purportedly act through a different anti-glycolytic mechanism. The ability to use the lowest possible effective dose of BHT and/or BHA can be a desirable outcome based on extensive dosage, safety, and efficacy studies. While a glucose anti-metabolite can be considered robust and comprehensive calorie restriction mimetics, improving their efficacy and/or cost-effectiveness may possibly be achieved. It is thought that by combining it with potentiating agents, such as BHT and/or BHA, which provide similar benefits but act through different metabolic mechanisms, such improvement of efficacy can be achieved. Thus, BHT and/or BHA represent potential agents that can be combined with a glucose anti-metabolite in a calorie restriction mimetic system to create a "coarse" and/or "fine" tuning adjustment in the metabolic effects and whole-body health benefits of glucose anti-metabolites.

Moreover, one of the major hallmarks of the calorie restriction phenotype is an increase in insulin sensitivity. This sensitivity is often accompanied by reduced plasma levels of insulin and also serves as an anti-diabetic mechanism. Moreover, the insulin signaling pathway (or its homolog in lower animal models) appears to be closely linked with longevity and responsive to many interventions which prolong it to maintain functionality and vitality. Mannoheptulose is one calorie restriction mimetic compound that has been shown to both lower plasma insulin levels and/or increase insulin sensitivity in mice, dogs, and other species.

In a possible complementary way, Moustafa et al (1995) demonstrated that BHT fed to rats for 18 months at a dosage of 140 mg/Kg body weight per day increased insulin sensitivity and partially ameliorated the age-related reduction in insulin-stimulated glucose transport in adipocytes. This beneficial effect may be due to its ability to protect cell membrane lipids from oxidative damage caused by free radicals. Damage from free radicals is a well known causative factor in the deterioration of many functions associated with the aging process. Although mannoheptulose is thought to exert some indirect protection against oxidative damage (see WO2008093302A2), its primary beneficial effect is on insulin sensitivity that likely results from the inhibition of hexokinase and glycolysis, thereby reducing overall glycolytic flux. Interestingly, Rady et al (1980) had previously shown no effect of BHT on hexokinase or other glycolytic enzymes in extracts of mouse lung. Therefore, taken together, the above findings suggest that BHT may act synergistically with glucose anti-metabolites to protect the mammalian body against age-related loss of insulin sensitivity by working through different, but complementary, molecular mechanisms.

Also, alloxan has been widely used in both *in vivo* and *in vitro* situations to induce diabetes. The alloxan-induced diabetic response is somewhat analogous to the elevated circulating insulin levels commonly observed during normal aging. Alloxan has negative effects on pancreatic beta cells due presumably to its role in generating free radicals, which result in increased insulin secretion. Maechler et al (1999) reported that BHA blocked the negative effects of alloxan using cultured insulin secreting cells. Most relevant to the beneficial effects of mannoheptulose for lowering plasma insulin levels is the ability of BHA to prevent the alloxan-induced increase in insulin secretion. While BHT appears to protect against the loss of insulin sensitivity, the benefits of BHA appear to be through its ability to protect cell membranes from free radical damage. BHT may also provide protection against elevated insulin levels. Thus, it is possible that BHA can work synergistically with a glucose anti-metabolite to prevent elevation of insulin levels by a different, but complementary, mechanism.

In summary, a glucose anti-metabolite, BHT, and BHA provide similar beneficial effects but act through different molecular processes and mechanisms. The differential modes of action of these compounds suggest that a combination of mannoheptulose, BHT, and/or BHA may have synergistic effects within the mammalian body to provide additional improvements in efficacy and potency. Thus, the addition of BHT and/or BHA in glucose anti-metabolite based calorie restriction mimetic formulations might be expected to result in greater insulin sensitivity and better protection against the age-related loss of insulin sensitivity and/or elevation of circulating insulin levels, while possibly lowering the concentration of mannoheptulose required to achieve its beneficial effects.

Thus, it would be beneficial to provide nutrition such as a glucose anti-metabolite in combination with BHA and/or BHT, specifically for companion animals. Accordingly, embodiments of the invention relate to such a composition.

SUMMARY

The invention is defined as in claim 1. One embodiment relates to a pet Food composition comprising a glucose anti-metabolite and BHA. The glucose anti-metabolite can comprise mannoheptulose. The BHA can be present at from about 2 mg per kg composition to about 140 mg per kg composition, or from about 6 mg per kg composition to about 80 mg per kg composition, or from about 10 mg per kg composition to about 60 mg per kg composition. The glucose anti-metabolite can be present in the composition at less than about 5% by weight of the composition. The pet food composition can be a wet composition, semi-moist composition, dry composition, and combinations thereof. The composition is a nutritionally balanced pet food composition.

Another embodiment relates to a pet food composition comprising a glucose anti-metabolite and BHT. The glucose anti-metabolite can comprise mannoheptulose. The BHT can be present at from about 2 mg per kg composition to about 140 mg per kg composition, or from about 6 mg per kg composition to about 80 mg per kg composition, or from about 10 mg per kg composition to about 60 mg per kg composition. The glucose anti-metabolite can be present in the composition at less than about 5% by weight of the composition. The composition can be a wet composition, semi-moist composition, dry composition, and combinations thereof. The composition is a nutritionally balanced pet food composition

Another embodiment relates to a pet food composition comprising a glucose anti-metabolite and BHA and BHT. The glucose anti-metabolite can comprise mannoheptulose. The BHA and BHT can be present combined at from about 2 mg per kg composition to about 140 mg per kg composition. The glucose anti-metabolite can be present in the composition at less than about 5% by weight of the composition. The composition can be a wet composition, semi-moist composition, dry composition, and combinations thereof. The composition is a nutritionally balanced pet food composition.

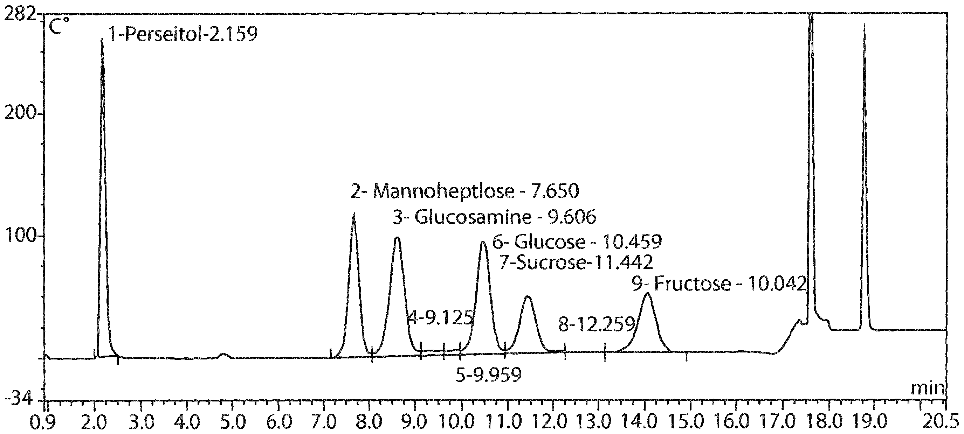

BRIEF DESCRIPTION OF THE DRAWING

The figure is an integrated amperometry waveform produced by the glucose anti-metabolite method.

DETAILED DESCRIPTION

Definitions

As used herein, the articles including "the", "a", and "an", when used in a claim or in the specification, are understood to mean one or more of what is claimed or described.

As used herein, the terms "include", "includes", and "including" are meant to be non-limiting.

As used herein, the term "plurality" means more than one.

As used herein, the terms "animal" or "pet" mean a domestic animal including, but not limited to domestic dogs (canines), cats (felines), horses, cows, ferrets, rabbits, pigs, rats, mice, gerbils, hamsters, horses, and the like. Domestic dogs and domestic cats are particular examples of pets and are referred to herein as "companion animals." It should be understood that throughout this disclosure when using the term animal, pet, or companion animal, the animal, pet, or companion animal is in a non-diseased state, unless otherwise stated.

As used herein, the terms "animal feed", "animal feed compositions", "animal feed kibble", "pet food", or "pet food composition" all mean a composition intended for ingestion by a pet. Pet foods can include, without limitation, nutritionally balanced compositions suitable for daily feed, as well as supplements and/or treats, which may or may not be nutritionally balanced.

As used herein, the term "nutritionally balanced" means that a composition, such as pet food, has known required nutrients to sustain life in proper amounts and proportions based on recommendations of recognized authorities, including governmental agencies, such as, but not limited to, Unites States Food and Drug Administration's Center for Veterinarian Medicine, the American Feed Control Officials Incorporated, in the field of pet nutrition, except for the additional need for water.

All oral doses of the invention are calculated per kilogram of body weight of the companion animal unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

All lists of items, such as, for example, lists of ingredients, are intended to and should be interpreted as Markush groups. Thus, all lists can be read and interpreted as items "selected from the group consisting of" ... list of items ... "and combinations and mixtures thereof."

Referenced herein are trade names for components including various ingredients utilized in embodiments of the invention. The inventors herein do not intend to be limited by materials under a certain trade name. Equivalent materials (e.g., those obtained from a different source under a different name or reference number) to those referenced by trade name may be substituted and utilized in the descriptions herein.

The processes, methods, compositions, and apparatuses herein may comprise, consist essentially of, or consist of any of the features or embodiments as described herein.

In the description of the various embodiments of the disclosure, various embodiments or individual features are disclosed. As will be apparent to the ordinarily skilled practitioner, all combinations of such embodiments and features are possible and can result in preferred executions of the disclosure. While various embodiments and individual features of the invention have been illustrated and described, various other changes and modifications can be made without departing from the spirit and scope of the invention. As will also be apparent, all combinations of the embodiments and features taught in the foregoing disclosure are possible and can result in preferred executions of the invention.

Embodiments of the invention

Embodiments of the invention relate to compositions comprising BHA and/or BHT and a glucose anti-metabolite component selected from the group consisting of 2-deoxy-D-glucose; 5-thio-D-glucose; 3-O-methylglucose; 1,5-anhydro-D-glucitol; 2,5-anhydro-D-glucitol; 2,5-anhydro-D-mannitol; mannoheptulose; and mixtures and combinations thereof. Without intending to be limited by theory, these components are accepted to be glucose anti-metabolites. In another embodiment, the components may be present in the recited compositions by virtue of a component of plant matter such as avocado, or other enriched source of mannoheptulose such as alfalfa, fig, primrose, and the like.

Glucose anti-metabolites

The glucose anti-metabolite components as disclosed herein include 2-deoxy-D-glucose, 5-thio-D-glucose, 3-O-methylglucose, anhydrosugars including 1,5-anhydro-D-glucitol, 2,5-anhydro-D-glucitol, and 2,5-anhydro-D-mannitol, mannoheptulose, and mixtures and combinations thereof. Mannoheptulose is one particular glucose anti-metabolite. In one embodiment, mannoheptulose may be present in the recited compositions as a component of plant matter such as an avocado, avocado extract, avocado meal, avocado concentrate, or other enriched source of mannoheptulose. Non-limiting examples of enriched sources of mannoheptulose include alfalfa, fig, or primrose. The plant matter may include the fruit, seed (or pit), branches, leaves, or any other portion of the relevant plant or combinations thereof.

Avocado (also commonly referred to as alligator pear, aguacate, or palta) contains unusually enriched sources of mannoheptulose, as well as related sugars and other carbohydrates. Avocado is a sub-tropical evergreen tree fruit, growing most successfully in areas of California, Florida, Hawaii, Guatemala, Mexico, the West Indies, South Africa, and Asia.

Species of avocado include, for example, Persea Americana and Persea nubigena, including all cultivars within these illustrative species. Cultivars may include 'Anaheim,' 'Bacon,' 'Creamhart,' 'Duke,' 'Fuerte,' 'Ganter,' 'Gwen,' 'Hass,' 'Jim,' 'Lula,' 'Lyon,' 'Mexicola Grande,' 'Murrieta Green,' 'Nabal,' 'Pinkerton,' 'Queen,' 'Puebla,' 'Reed,' 'Rincon,' 'Ryan,' 'Spinks,' 'Topa Topa,' 'Whitsell,' 'Wurtz,' and 'Zutano.' The fruit of the avocado is particularly preferred for use herein, which may contain the pit or wherein the pit is removed or at least partially removed. Fruit from Persea Americana is particularly preferred for use herein, as well as fruit from cultivars which produce larger fruits (e.g., about 12 ounces or more when the fruit is mature), such as Anaheim, Creamhart, Fuerte, Hass, Lula, Lyon, Murrieta Green, Nabal, Queen, Puebla, Reed, Ryan and Spinks.

Plant matter from alfalfa, fig, or primrose is also reported to provide relatively high levels of mannoheptulose. Alfalfa is also referred to as Medicago sativa. Fig or Ficus carica (including Cluster fig or Sycamore fig, for example) may also be used, as well as primrose or Primula officinalis.

It has been discovered that particular levels of a component selected from 2-deoxy-D-glucose; 5-thio-D-glucose; 3-O-methylglucose; 1,5-anhydro-D-glucitol; 2,5-anhydro-D-glucitol; 2,5-anhydro-D-mannitol; mannoheptulose; and mixtures and combinations thereof can be useful herein. In particular, it has been found that relatively low levels, as well as relatively high doses of the component, while useful, may provide less than optimal efficacy for desired purposes. Dosage will depend upon the glucose anti-metabolite component used and will vary depending upon the size and condition of the companion animal to which the glucose anti-metabolite is to be administered. Dosage in the range of about 0.0001 or about 0.001 grams/kg to about 1 g/kg can be beneficial in some embodiments. As used herein, when dosage in mg/kg is used, the "mg" refers to the level of the component, such as mannoheptulose, and "kg" refers to kilograms of body weight of the companion animal, such as a dog or cat. Dosage at the lower range may also be appropriate when using 2-deoxy-D-glucose in large animals. Higher dosage, particularly of compounds such as 5-thio-D-glucose or 2,5-anhydro-D-mannitol, may also be readily tolerated. In one embodiment, the dosage of the component provided to a companion animal on a daily basis may be from about 0.1, 0.5, 1, 2, or 5 mg/kg to about 15, 20, 50, 100, 150, or 200 mg/kg, and all combinations of these ranges, wherein "mg" refers to the level of the component and "kg" refers to kilograms of body weight of the companion animal. In one embodiment, the dosage to the companion animal, on a daily basis, may be from about 1 mg/kg to about 15 mg/kg, from about 2 mg/kg to about 10 mg/kg, or from about 2 mg/kg to about 5 mg/kg. In one embodiment, the dosage to the companion animal, on a daily basis, may be from about 1 mg/kg to about 5 mg/kg, from about 1.5 mg/kg to about 5 mg/kg, from about 2 mg/kg to about 5 mg/kg, or about 2 mg/kg. In certain embodiments, these amounts may translate to compositions comprising less than about 5%, or less than about 2%, or from about 0.0001% to about 0.5%, or from about 0.1% to about 10%, or from about 0.1% to about 5%, of the component, all by weight of the composition. All ranges therebetween are envisioned. The level of component may be determined by one of ordinary skill in the art based on a variety of factors, for example, the form of the composition (e.g., whether a dry composition, semi-moist composition, wet composition, or supplement, or any other form or mixture thereof). The ordinarily skilled artisan will be able to utilize the preferred dosage and determine the optimal level of component within a given composition.

Similarly, the overall dosage amount of the component on a daily basis provided to the companion animal may be provided. Such a daily dosage amount can be from about 0.1 mg per day to about 1000 mg per day. Such daily dosage amounts can be dependent on the size of the companion animal consuming the composition. For example, in one embodiment, larger companion animals may consume more than smaller companion animals. Of course, that is consistent with the dosing disclosed herein with respect to a dosing amount per mass of the companion animal. Thus, in one embodiment, as the companion animal increases in size, more of the composition can be administered.

Accordingly, in one embodiment, such a daily dosage amount can correspond to the dosage on a daily basis per mass of the companion animal, as described herein. Specifically, daily dosage amounts can range, in some embodiments, from about 0.1 mg per day to about 1000 mg per day, or even more, depending on the size of the companion animal and the daily dosage amounts as described above. In other embodiments, the daily dosage can be from about 1 mg per day to about 500 mg per day, or from about 1 mg per day to about 200 mg per day, or from about 1 mg per day to about 100 mg per day, or from about 5 mg day per day to about 100 mg per day, or from about 5 mg per day to about 80 mg per day, or from about 10 mg per day to about 50 mg per day, or about 40 mg per day. All ranges therebetween are also envisioned.

Similarly, wherein an extract or meal of plant matter is utilized in the compositions herein, levels of extract or meal may be dependent upon level of efficacious component within such extract or meal. Extracts and/or meals have been found herein which comprise from about 0.5% to about 99% of the glucose anti-metabolite component, alternatively from about 0.5% to about 75% of the glucose anti-metabolite component, alternatively from about 0.5% to about 50% of the glucose anti-metabolite component, alternatively, from about 0.5% to about 25% of the glucose anti-metabolite component, all by weight of the extract or meal. Extracts and/or meals have been found herein in which the glucose anti-metabolite component may be from about 0.5, 1, 2, 5, or 10% to about 15, 25, 50 or 75% by weight of the extract and/or meal.

BHA and BHT

As described, the composition of embodiments of the present invention can comprise BHA and/or BHT. BHA and BHT are synthetic antioxidants that can be applied to fat and fatty foods to prevent oxidative deterioration. Since they are synthetic and do not occur naturally, the compositions of the present invention related to embodiments comprising synthetic BHA and/or BHT.

In one embodiment, only BHA is included (exclusive of BHT) in compositions of the present invention. In other embodiments, both BHA and BHT are included in compositions of the present invention.

BHA and/or BHT can be present in the composition through any number of sources. In one embodiment, BHA and/or BHT can be included by addition via fat during the preparation of the composition. BHA and/or BHT can typically be used as food additives in fat to prevent or reduce oxidation. In other embodiments BHA and/or BHT can be added to the compositions herein through the addition of other carriers.

The composition can include varying amounts of BHA and/or BHT. Each of these is addressed as follows.

In one embodiment, BHA can be included in the compositions herein by amount of diet. Thus, the total amount of BHA in the compositions can be in one embodiment from about 2 to about 140 mg/kg diet. In other embodiments, the total amount of BHA can be present at from about 3 to about 120 mg/kg diet, or from about 4 to about 100 mg/kg diet, or from about 5 to about 90 mg/kg diet, or from about 6 to about 80 mg/kg diet. In one embodiment, the total amount of BHA can be present at from about 10 to about 30 mg/kg diet.

BHA can be included in the compositions herein by amount administered to the animal per day. Thus, the total amount of BHA administered to the animal per day can be in one embodiment from about 0.12 to about 210 mg/day. In other embodiments, the total amount of BHA administered to the animal per day can be from about 0.13 to about 200 mg/day, or from about 0.14 to about 180 mg/day, or from about 0.15 to about 160 mg/day, or from about 0.16 to about 140 mg/day, or from about 0.2 to about 120 mg/day. In one embodiment, the total amount of BHA administered to the animal per day can be from about 1 to about 20 mg/day.

BHA can be included in the compositions herein by amount administered to the animal per day per body weight of the animal. Thus, the total amount of BHA administered to the animal per day per body weight of the animal can be in one embodiment from about 0.04 to about 3.6 mg/day/kg body weight. In other embodiments, the total amount of BHA administered to the animal per day per body weight of the animal can be from about 0.06 to about 3 mg/day/kg body weight, or from about 0.08 to about 2.5 mg/day/kg body weight, or from about 0.08 to about 2.25 mg/day/kg body weight, or from about 0.1 to about 2 mg/day/kg body weight. In one embodiment, the total amount of BHA administered to the animal per day per body weight of the animal can be from about 0.1 to about 0.6 mg/day/kg body weight.

In one embodiment, BHT can be included in the compositions herein by amount of diet. Thus, the total amount of BHT in the compositions can be in one embodiment from about 2 to about 140 mg/kg diet. In other embodiments, the total amount of BHT can be present at from about 3 to about 120 mg/kg diet, or from about 4 to about 100 mg/kg diet, or from about 5 to about 90 mg/kg diet, or from about 6 to about 80 mg/kg diet. In one embodiment, the total amount of BHT can be present at from about 10 to about 30 mg/kg diet.

BHT can be included in the compositions herein by amount administered to the animal per day. Thus, the total amount of BHT administered to the animal per day can be in one embodiment from about 0.12 to about 210 mg/day. In other embodiments, the total amount of BHT administered to the animal per day can be from about 0.13 to about 200 mg/day, or from about 0.14 to about 180 mg/day, or from about 0.15 to about 160 mg/day, or from about 0.16 to about 140 mg/day, or from about 0.2 to about 120 mg/day. In one embodiment, the total amount of BHT administered to the animal per day can be from about 1 to about 20 mg/day

BHT can be included in the compositions herein by amount administered to the animal per day per body weight of the animal. Thus, the total amount of BHT administered to the animal per day per body weight of the animal can be in one embodiment from about 0.04 to about 3.6 mg/day/kg body weight. In other embodiments, the total amount of BHT administered to the animal per day per body weight of the animal can be from about 0.06 to about 3 mg/day/kg body weight, or from about 0.08 to about 2.5 mg/day/kg body weight, or from about 0.08 to about 2.25 mg/day/kg body weight, or from about 0.1 to about 2 mg/day/kg body weight. In one embodiment, the total amount of BHT administered to the animal per day per body weight of the animal can be from about 0.1 to about 0.6 mg/day/kg body weight.

As described, in one embodiment, only BHA can be present (exclusive of BHT) in compositions of the present invention. That is to say, BHA may be present but not BHT in compositions of the present invention. In other embodiments, both BHA and BHT are included in compositions of the present invention.

As described, in one embodiment, only BHT can be present (exclusive of BHA) in compositions of the present invention. That is to say, BHT may be present but not BHA in compositions of the present invention. In other embodiments, both BHA and BHT are included in compositions of the present invention.

Any combination BHA and BHT can be included in the compositions herein by amount of diet. Thus, the total amount of BHA and BHT in the compositions can be in one embodiment from about 2 to about 140 mg/kg diet. In other embodiments, the total amount of BHA and BHT can be present at from about 3 to about 120 mg/kg diet, or from about 4 to about 100 mg/kg diet, or from about 5 to about 90 mg/kg diet, or from about 6 to about 80 mg/kg diet. In one embodiment, the total amount of BHA and BHT can be present at from about 10 to about 30 mg/kg diet.

Any combination BHA and BHT can be included in the compositions herein by amount administered to the animal per day. Thus, the total amount of BHA and BHT administered to the animal per day can be in one embodiment from about 0.12 to about 210 mg/day. In other embodiments, the total amount of BHA and BHT administered to the animal per day can be from about 0.13 to about 200 mg/day, or from about 0.14 to about 180 mg/day, or from about 0.15 to about 160 mg/day, or from about 0.16 to about 140 mg/day, or from about 0.2 to about 120 mg/day. In one embodiment, the total amount of BHA and BHT administered to the animal per day can be from about 1 to about 20 mg/day.

Any combination BHA and BHT can be included in the compositions herein by amount administered to the animal per day per body weight of the animal. Thus, the total amount of BHA and BHT administered to the animal per day per body weight of the animal can be in one embodiment from about 0.04 to about 3.6 mg/day/kg body weight. In other embodiments, the total amount of BHA and BHT administered to the animal per day per body weight of the animal can be from about 0.06 to about 3 mg/day/kg body weight, or from about 0.08 to about 2.5 mg/day/kg body weight, or from about 0.08 to about 2.25 mg/day/kg body weight, or from about 0.1 to about 2 mg/day/kg body weight. In one embodiment, the total amount of BHA and BHT administered to the animal per day per body weight of the animal can be from about 0.1 to about 0.6 mg/day/kg body weight.

In one embodiment, the ratio of BHA to BHT can be about 1:1. In other embodiments, any ratio can be used, such as 0:1 or 1:0.

Compositions

Accordingly, embodiments of the invention are directed to a composition that is intended for ingestion by a companion animal and that comprises a glucose anti-metabolite and BHA and/or BHT, as described herein. Compositions include foods intended to supply necessary dietary requirements, as well as treats (e.g., biscuits) or other food supplements. Optionally, the composition herein may be a dry composition (for example, kibble), semi-moist composition, wet composition, or any mixture thereof. Alternatively or additionally, the composition is a supplement, such as a gravy, drinking water, yogurt, powder, suspension, chew, treat (e.g., biscuits) or any other delivery form.

Moreover, the composition is nutritionally balanced, such as a pet food kibble. In a comparative example, the composition is not nutritionally balanced, such as a supplement, treat, or other delivery form for a pet. Nutritionally balanced pet foods and supplements, and the manufacturing processes thereof, are well known in the art.

The compositions used herein may optionally comprise one or more further components. Other components are beneficial for inclusion in the compositions used herein, but are optional for purposes of the invention. In one embodiment, the compositions may comprise, on a dry matter basis, from about 10% to about 90% crude protein, alternatively from about 20% to about 50% crude protein, alternatively from about 20% to about 40% crude protein, by weight of the composition, or alternatively from about 20% to about 35% crude protein, by weight of the composition. The crude protein material may comprise vegetable-based proteins such as soybean, cereals (corn, wheat, etc), cottonseed, and peanut, or animal-based proteins such as casein, albumin, and meat protein. Non-limiting examples of meat protein useful herein include a protein source selected from the group consisting of beef, pork, lamb, poultry, fish, and mixtures thereof.

Furthermore, the compositions may comprise, on a dry matter basis, from about 5% to about 40% fat, alternatively from about 10% to about 35% fat, by weight of the composition.

Embodiments related to compositions of the invention may further comprise a source of carbohydrate. In one embodiment, the compositions may comprise from about 35%, by weight of the composition, up to about 50%, by weight of the composition, carbohydrate source. In other embodiments, the composition can comprise from about 35% to about 45%, by weight of the composition, or from about 40% to 50%, by weight of the composition, carbohydrate source. Grains or cereals such as rice, corn, milo, sorghum, barley, wheat, and the like are illustrative sources of carbohydrate.

The compositions may also contain other materials such as, but not limited to, dried whey and other dairy by-products, beet pulp, cellulose, fiber, fish oil, flax, vitamins, minerals, flavors, antioxidants, and taurine.

The compositions may also contain other optional ingredients. Optional ingredients can include Probiotic components (Bifidobacteria and/or Lactobacillus) and Prebiotic (fructooligosaccharides) components. Examples and amounts of Probiotic components and Prebiotic components that can be included are disclosed in United States Publication No. 2005/0158294, for example. Other optional ingredients that can be included are omega 6 and omega 3 fatty acids, carnitine, hexametaphosphate, glucosamine, chondroitin sulfate, carotenoids including beta carotene, vitamin E, and lutein, and those ingredients as shown in Table 1 below.

Examples

The following examples are provided to illustrate embodiments of the invention and are not intended to limit the scope thereof in any manner.

Preparation of Mannoheptulose-containing Avocado Meal

Fresh avocados (Lula variety) were obtained from Fresh King Incorporated (Homestead, FL). The avocados were manually split open and the pits were removed and discarded. The remaining skin and pulp were ground through a Hobart Commercial Food Preparation machine (Serial No. 11-10410235) using a 12 ¼ sieve. The ground avocado was then transferred to an Edwards Freeze Drier (Super Modulyo Model, Crawely, Sussex, England). The freeze drier was set at -20 °C for the first 24 hours, -5 °C for the following 24 hours and 5 °C for the final 72 hours. Upon removal from the freeze drier, the meal was ground to a powder using a Straub Grinding Mill (model 4E, Philadelphia, PA). The avocado meal was analyzed and found to contain about 10.35% mannoheptulose, by weight of the meal. It should be noted that the amount of mannoheptulose found in avocados varies with the particular strain and state of ripeness.

Preparation of Avocado Extract

Avocado extract containing enhanced levels of mannoheptulose is prepared in accordance with the following optional process and utilized in compositions of embodiments of the invention.

Whole avocado fruit (about 900 kilograms) is provided. The fruit is split and the pits are removed, either partially or wholly, providing about 225 kilograms of pitted avocado halves. The raw avocado is charged to a disintegrator, whereupon some agitation, water (about 3000 kilograms) and CELLUBRIX (commercially available from Novozymes A/S) (about 1 liter) is further charged. The mixture is further agitated and concurrently heated to about 66 °C. Upon completion of the charge, further CELLUBRIX (about 1 liter) is added, and the entire mixture is held under agitation for about 12 hours at a controlled pH of about 5.5. The temperature is then further increased to about 80 °C and then held for at least about 2 hours. The resulting digested plant mixture is then filtered at 80 °C to provide the carbohydrate extract as the filtrate. The carbohydrate extract is then evaporated in a simplified recirculation system at 80 °C, under vacuum, to provide the carbohydrate extract having from about 10% to about 20% solids and a pH of about 5.5. The extract is then further concentrated using a refractance window dryer to provide about 100 kilograms of the extract as a crystalline or powder (a yield of about 11% carbohydrate extract, based on the starting mass of the whole avocado fruit, which is analyzed as a yield from about 0.25% to about 4.5% mannoheptulose, based on the starting mass of the whole avocado fruit). It should be noted the amount of mannoheptulose found in avocados varies with the particular strain and state of ripeness of the fruit. The extract may be used in the compositions of embodiments of the invention.

Kibbles

Table 1 illustrates two kibble compositions having the following components at the approximate indicated amounts that can be prepared using processes that are standard in the art, including extrusion, and that can be fed to dogs and/or cats as a daily feed:

Table 1

| Component | Diet 1: Component Amount indicated as Wt% (unless noted) | Diet 2: Component Amount indicated as Wt% (unless noted) |
|---|---|---|
| Extract of Avocado* | 0.02 | 0.01 |
| Chicken, Chicken By-product Meal, Fish Meal, and Egg | 44 | 47 |
| Chicken Fat | 8 | 6 |
| Beet Pulp | 2 | 3 |
| Salts | 2.5 | 2 |
| Vitamins and Minerals** | 1 | 1 |
| Minors*** | 3.5 | 4 |
| BHT | 6 mg/kg diet | 0 mg/kg diet |
| BHA | 6 mg/kg diet | 24 mg/kg diet |
| Grains (corn, sorghum, barley, rice, wheat) | Remainder | Remainder |

*Avocado may be substituted with other plant mailer having enhanced mannoheptulose content. The incorporation of a mannoheptulose source likely replaces a similar amount of a grain source in the composition.
**Vitamins and Minerals may include: Vitamin E, beta-carotene, Vitamin A, Ascorbic Acid, Calcium Pantothenate, Biotin, Vitamin $B_{12}$, Vitamin $B_1$, Niacin, Vitamin $B_2$, Vitamin $B_6$, Vitamin $D_3$, Vitamin $D_2$, Folic Acid, Choline Chloride, Inositol, Calcium Carbonate, Dicalcium Phosphate, Potassium Chloride, Sodium Chloride, Zinc Oxide, Manganese Sulfate, Copper Sulfate, Manganous Oxide, Ferrous Sulfate, Potassium Iodide, Cobalt Carbonate.
***Minors may include: Fish oil, flax seed, flax meal, cellulose, flavors, antioxidants, taurine, yeast, carnitine, chondroitin sulfate, glucosamine, lutein, rosemary extract.

Administration

Eighty (n=80) Labrador Retrievers can be randomized by age, gender, and littermate to receive either a complete and nutritionally balanced control diet that is similar to Eukanuba® Senior Large Breed or an experimental diet that is identical to the control diet except for the inclusion of mannoheptulose and BHA and BHT as disclosed below. The dogs can be split into two study groups.

Study 1: A total of 39 older Labrador Retrievers can be fed a nutritionally-balanced composition providing mannoheptulose at levels of 0 or about 2 mg/kg of body weight of the dog and BHA and BHT at 0 mg per kg diet or about 12 mg per kg diet, respectively. Average age of the dogs (12 neutered males, 27 spayed females) at the start of a 4-year study is 6.7 years with a range of 5.1 to 8.2 years of age for the youngest and oldest dog within the cohort, respectively. The control composition can be fed as a nutritionally-balanced composition, and it contains no mannoheptulose (0 mg/kg), BHA and BHT (0 mg per kg diet), avocado extract, avocado meal, or avocado concentrate. The test composition can be the nutritionally-balanced control composition formulated with avocado extract, avocado meal, or avocado concentrate to provide mannoheptulose at a dose of about 2 mg/kg body weight of the dog and BHA and BHT at about 12 mg per kg diet. Older dogs can be fed one-half their daily allotment of food at 0730 and 1430 each day. Dogs can be fed to maintain body weight and body composition score (BCS) within a 2-4 score range. If food adjustments were to be made, they should be made on a quarterly basis. All dogs can be fasted overnight, and morning meals can be withheld until blood collections could be conducted for all immune measurements. Water is provided ad lib.

Study 2: A total of 41 younger Labrador Retrievers can be fed a nutritionally-balanced composition providing mannoheptulose at levels of 0 or about 2 mg/kg of body weight of the dog and BHA at 0 mg per kg diet or about 24 mg per kg diet, respectively. Average age of the dogs (12 neutered males, 29 spayed females) at the start of the 36-month feeding study is 4.0 years with a range of 2.0 to 6.1 years of age for the youngest and oldest dog within the cohort, respectively. The control composition can be fed as a nutritionally-balanced composition (Eukanuba® Senior Maintenance Formula), and it contains no mannoheptulose (0 mg/kg), BHA and BHT (0 $\mu$g per g diet), avocado extract, avocado meal, or avocado concentrate. The test composition can be the nutritionally-balanced control composition formulated with avocado extract, avocado meal, or avocado concentrate to provide mannoheptulose at a dose of about 2 mg/kg body weight of the dog and BHA at about 24 mg per kg diet. Younger dogs can be fed one-half their daily allotment of food at 0730 and 1430 each day. Dogs can be fed to maintain body weight and body composition score (BCS) within a 2-4 score range. If food adjustments were to be made, they should be made on a quarterly basis. However, all dogs can be fasted overnight, and morning meals can be withheld until blood collections could be conducted for all immune measurements. Water is provided ad lib.

Methods

The glucose anti-metabolite, such as mannoheptulose, can be measured in a pet food or supplement, as follows.
Procedure (use only deionized water):

Weigh approx. 0.1 g feed/ingredient into a 15 mL plastic centrifuge tube.
Add 10 mL water to the tube and shake for 5 min.
Centrifuge tube at max speed (2440 g) for 5 min.
Dispense some of the supernatant into a 0.2 $\mu$m nylon centrifuge filter and spin at max speed (14000 g) for 5 min.
The sample is ready for injection.

Prepare a 10 $\mu$g/ml carbohydrate standard by dissolving 10 mg of each carbohydrate in 1 L water.
Prepare a 1 $\mu$g/ml carbohydrate standard by dissolving 100 $\mu$l of the 10 $\mu$g/ml solution in 900 $\mu$l water.
Prepare a 0.1 $\mu$g/ml carbohydrate standard by dissolving 10 $\mu$l of the 10 $\mu$g/ml solution in 990 $\mu$l water.
IC conditions: Eluent clean-up: Ionpac ATC-3 (Dionex P/N 059661), Boratetrap (Dionex P/N 047078). Column: CarboPac PA20 (Dionex P/N 060142), 2 mm Aminotrap precolumn (Dionex P/N 046122). Column Temperature: 30°C

PUMP

Flow: 0.4 ml/min
Eluents: A=Water, B=0.2 M NaOH, D=1 M NaOH.

| 0 min | 4%B | 0%C | 0%D |
|---|---|---|---|
| 14 min | 4%B | 0%C | 0%D |
| 14.01 min | 4%B | 0%C | 40%D |
| 20 min | 4%B | 0%C | 40%D |
| 20.01 min | 4%B | 0%C | 0%D |
| 30.0 min | 4%B | 0%C | 0%D |

Note: It may be necessary to regenerate the column before use with a 30-60 min flush with 1 M NaOH, followed by a 30-60 min rinse with 95% water: 5% 0.2M NaOH. Follow the recommended procedure from Dionex to prepare eluents.

AUTOSAMPLER

Injection Volume: 10 μl full loop

INTEGRATED AMPEROMETRY WAVEFORM

| | |
|---|---|
| Time = 0 | Potential = 0.1 |
| Time = 0.2 | Potential = 0.1, Begin Integration |
| Time = 0.4 | Potential = 0.1, End Integration |
| Time = 0.41 | Potential = -2 |
| Time = 0.42 | Potential = -2 |
| Time = 0.43 | Potential = 0.6 |
| Time = 0.44 | Potential = -0.1 |
| Time = 0.5 | Potential = -0.1 |

NOTE: Quantitate all peaks using peak areas. An example of an integrated amperometry waveform can be seen in the figure.

References:

1. Shaw, P.E.; Wilson, C.W.; Knight, R.J. J. Agric. Food Chem. 1980, 28, 379-382.

2. Dionex CarboPac20 Document No. 031844-01.

BHA and/or BHT

Extract crude fat as described in AOAC official method 954.02 using acid hydrolysis and determine the BHA and/or BHT by AOAC official method 983.15: Phenolic Antioxidants in Oils, Fats, and Butter Oil, as follows.

Place ca 2 g, accurately weighed, ground, well-mixed test portion in Mojonnier fat-extraction tube, add 2 mL alcohol to prevent lumping on addition of acid, and shake to moisten all particles. Add 10 mL HCl (25 + 11), mix well, and set tube 30-40 min in water bath at 70°-80°C, shaking frequently. Cool to room temperature and add alcohol until liquid level rises into constricted portion of Mojonnier tube.

Add 25 mL ether, stopper with glass, Neoprene, or good quality rubber stopper thoroughly cleaned with alcohol, and shake vigorously 1 min. Carefully release pressure so that no solvent is lost. Wash adhering solvent and fat from stopper back into extraction tube with few mL redistilled petroleum ether (bp <60°C).

Add 25 mL redistilled petroleum ether, stopper, and shake vigorously 1 min. Let stand until upper liquid is practically clear or centrifuge 20 min at ca 600 rpm. Pour as much of ether-fat solution as possible through filter consisting of cotton pledget packed just firmly enough in funnel stem to let ether pass freely into 150 mL beaker containing several glass beads. Rinse lip of tube with few mL petroleum ether. Reextract liquid remaining in tube twice, each time with only 15 mL of each ether, shaking 1 min after addition of each ether. Pour clear ether solution through filter into same beaker as before, and wash tip of tube, stopper, funnel, and end of funnel stem with few mL of mixture of 2 ethers (1 + 1).

Evaporate slowly on steam bath under gentle stream of air or $N_2$. Continue heating on steam bath 15 min after solvent has evaporated; then cool to room temperature.

Redissolve dried fat residue in four 10 mL portions ethyl ether, filtering each portion through small pledget of cotton into 100 mL beaker, containing few glass beads, that has been predried 30 min at 100°C, cooled to room temperature in desiccator, and weighed immediately. Use fifth 10 mL portion ether for rinsing cotton and funnel. Evaporate ether on steam bath, dry 90 min at 100°C, cool to room temperature in desiccator, and weigh immediately. Correct this weight by blank determination on reagents used.

The above extracted fat is subsequently used for BHA/BHT analysis as follows.

A. The BHA and/or BHT content of a feed can be measured by AOAC Official Method 983.15: Phenolic Antioxidants in Oils, Fats, and Butter Oil, as follows.

B. Apparatus:

 a. Gradient liquid chromatograph. With 10 mV recorder or integrator to electronically measure peak heights, 10 μL loop injection valve, and detector to measure absorbance at 280 nm. Typical operating conditions: detector sensitivity, 0.05 AUFS; temperature, ambient; flow rate, 2.0 m/min.
 b. LC column. Packed with C18-bonded spherical silica, or equivalent. Use guard column if desired.
 c. Glassware. Rinse all glassware with $CHCL_3$, acetone, and methanol, successively, and blow dry with $N_2$.

C. Reagents:

 a. Solvents. Acetonitrile, 2-propanol, and hexane. Distilled-in-glass grade.
 b. Mobile phase. (1) 5% acetic acid in $H_2O$ - LC grade. (2) Acetonitrile-methanol (1+1, v/v) - LC grade. Run linear gradient, from 30% of (2) in (1) to 100% (2), over 10 min with hold until last antioxidant (DG) is eluted. For test solution only, increase flow rate to 4 mL/min at 100% (2) over 6 min or until nonpolar lipids are eluted. For test solutions and standards, return to 30% (v/v) (2) in (1) over 1 min at 2 mL/min and let baseline and pressure stabilize (ca 6 min). Run blank solvent gradient (no injection) to ensure that no peaks interfering with any antioxidant are present. To remove or reduce peaks arising from elution solvent (1), replace inlet filter with prerinsed solid-phase C18 extraction cartridge and use in-line filter. If small interfering peaks are not eliminated, subtract peak height or gradient interference from that of relevant standard or test solution.
 c. Antioxidants. BHA (2- and 3-BHA mixture), BHT
 d. Standard solutions. Prepare in 2-propanol-acetonitrile (1+1, v/v). (1) Stock standard solution - 1 mg/mL. Accurately weight ca 50 mg to nearest 0.1 mg each antioxidant and transfer into single 50 ml volumetric flask. Dissolve, dilute to volume, and mix. (2) Working standard solution - 0.01 mg/ml. Pipet 1 mL stock standard solution into 100 mL volumetric flask, dilute to volume, and mix.
 e. Extraction solvents. (1) Standard hexane. Saturate ca 300 mL hexane in separatory funnel by adding acetonitrile until 2 layers remain after shaking 2 min. Discard acetonitrile lower layer. (2) Saturated acetonitrile. Saturate ca 300 mL acetonitrile in separatory funnel by adding hexane until 2 layers remain after shaking 2 min. Remove and discard hexane upper layer.

D. Determination:

 a. Extraction. Accurately weight to the nearest 0.01 g 50 mL beaker containing ca 5.5 g liquid or butter oil or ca 3.0 g lard or shortening (liquefied in bulk using 60C water bath or oven, and swirled or shaken to ensure homogeneity). Decant as much test portion as possible into 125 mL separatory funnel containing 20 mL (22.5 mL for lard or shortening) saturated hexane. Reweight beaker to determine test portion weight. Swirl to mix test portion with hexane, and extract with three 50 mL portions of saturated acetonitrile. If emulsions form, hold separatory funnel under hot tap water 5-10s. Collect extracts in 250 mL separatory funnel and let combined extracts slowly drain into 250 or 500 mL round-bottom flask to aid removal of hexane-oil droplets. (Note: At this point, 150 mL acetonitrile extract may be stored overnight, refrigerated).
 Evaporate to 3-4 mL, using flash evaporator with <= 40C water bath, within 10 min. Using disposable pipet, transfer acetonitrile-oil droplet mixture to 10 mL glass-stoppered graduated cylinder. Rinse flask with small portions nonsaturated acetonitrile. As rinse pools in flask bottom, pipet rinse to cylinder until 5 mL is collected. Rinse pipet through top and continue to rinse flask with small portions 2-propanol, transferring rinses to cylinder until 10 mL is collected. Mix cylinder contents.
 b. Chromatography. Using loop injection valve, inject 10 μL test extract and elute with solvent gradient program for test extracts, C(b). Before and after every 3-4 test injections, or more frequently if differences between standard peak heights are found to be>5%, inject 10 μL antioxidant working standard solution (10 μL/mL) and elute with solvent gradient program for standards, C,(b). For analyte peaks off scale or >3x standard, quantitatively dilute test extract with 2-propanol-acetonitrile (1+1) and reinject. Identify peaks by comparison with retention time of standard.
 For reagent blank determination, take 25 mL saturated hexane and follow extraction, (a), from "...extract with three 50 mL portions of saturated acetonitrile." Inject 10 μL. reagent blank extract and elute with solvent gradient program for analytes. The reagent blank should have no peaks interfering with antioxidant determination.
 Use electronically determined peak height, or measure peak height to 0.1 MM, using blank gradient chromatogram as guide to follow baseline. Determine antioxidant peak heights and average antioxidant standard peak heights (from duplicate injections before and after test injection, corrected for gradient blank).

E. Calculations:

Calculate concentration of antioxidant as follows: Antioxidant,

$$\mu g/g = (R_x/R_s) \times (C_s/W_x) \times D$$

where $R_x$ and $R_s$ are peak heights from test portion and standard, respectively; $C_s$ is concentration standard, $\mu g/mL$; $W_x$ is test portion weight, g/mL, in undiluted 10 mL test extract; and D is dilution factor, if solution injected is diluted.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention.

While particular embodiments of the invention have been illustrated and described, it would be obvious to those skilled in the art that various other changes and modifications can be made without departing from the scope of the invention, which is defined by the claims.

**Claims**

1. A pet food composition comprising a glucose anti-metabolite and BHA and/or BHT;
   wherein the glucose anti-metabolite is selected from the group consisting of 2-deoxy-D-glucose, 5- thio-D-glucose, 3-O-methylglucose, 1,5-anhydro-D-glucitol, 2,5-anhydro-D-glucitol, 2,5- anhydro-D-mannitol, mannoheptulose, and mixtures and combinations thereof; and
   wherein the pet food composition is nutritionally balanced.

2. The pet food composition of claim 1, wherein BHA is present at from 2 mg per kg composition to 140 mg per kg composition, or from 6 mg per kg composition to 80 mg per kg composition, or from 10 mg per kg composition to 60 mg per kg composition.

3. The pet food composition of claim 1, wherein BHT is present at from 2 mg per kg composition to 140 mg per kg composition, or from 6 mg per kg composition to 80 mg per kg composition, or from 10 mg per kg composition to 60 mg per kg composition.

4. The pet food composition of any of the preceding claims, wherein BHA and/or BHT are present, alone or in combination with one another, at from 2 mg per kg composition to 140 mg per kg composition, or from 3 mg per kg composition to 120 mg per kg composition, or from 4 mg per kg composition to 100 mg per kg composition, or from 5 mg per kg composition to 90 mg per kg composition, or from 6 mg per kg composition to 80 mg per kg composition, or from 10 mg per kg composition to about 30 mg per kg composition..

5. The pet food composition according to any of the preceding claims comprising BHA and BHT.

6. The pet food composition according to claim 5, wherein the ratio of BHA to BHT is about 1:1.

7. The pet food composition according to any of the preceding claims, wherein the glucose anti-metabolite is present in the pet food composition at less than about 5% by weight of the composition.

8. The pet food composition according to any of the preceding claims, wherein the glucose anti-metabolite comprises mannoheptulose.

9. The pet food composition according to any of the preceding claims, wherein the pet food composition is a composition for dogs or cats.

10. The pet food composition according to any of the preceding claims, wherein the pet food composition further comprises, on a dry matter basis, from 10% to 90% crude protein, or from 20% to 50% crude protein, or from 20% to 40% crude protein, or from 20% to 35% crude protein, by weight of the composition.

11. The pet food composition according to any of the preceding claims, wherein the pet food composition further comprises, on a dry matter basis, from 5% to 40% fat, by weight of the composition, or from 10% to 35% fat, by weight of the composition.

12. The pet food composition according to any of the preceding claims, wherein the pet food composition further comprises from 35% to 50%, by weight of the composition, a carbohydrate source, or from 35% to 45%, by weight of the composition, a carbohydrate source, or from 40% to 50%, by weight of the composition, a carbohydrate source.

13. The pet food composition of claim 12, wherein the carbohydrate source comprises rice, corn, milo, sorghum, barley, wheat and/or combinations thereof.

14. The pet food composition of any of the preceding claims, further comprising dried whey, dairy by-products, beet pulp, cellulose fiber, fish oil, flax, vitamins, minerals, flavors, antioxidants, and/or taurine.

15. Pet food composition according to any of the preceding claims for use in a method for treatment of the animal body by therapy.


**Patentansprüche**

1. Tiernahrungszusammensetzung, umfassend einen Glucose-Antimetaboliten und BHA und/oder BHT; wobei der Glucose-Antimetabolit ausgewählt ist aus der Gruppe, bestehend aus 2-Desoxy-D-Glucose, 5-Thio-D-Glucose, 3-O-Methylglucose, 1,5-Anhydro-D-Glucitol, 2,5-Anhydro-D-Glucitol, 2,5-Anhydro-D-Mannitol, Mannoheptulose und Mischungen und Kombinationen davon; und wobei die Tiernahrungszusammensetzung diätetisch ausgewogen ist.

2. Tiernahrungszusammensetzung nach Anspruch 1, wobei BHA mit 2 mg pro kg der Zusammensetzung bis 140 mg pro kg der Zusammensetzung, oder mit 6 mg pro kg der Zusammensetzung bis 80 mg pro kg der Zusammensetzung, oder mit 10 mg pro kg der Zusammensetzung bis 60 mg pro kg der Zusammensetzung vorliegt.

3. Tiernahrungszusammensetzung nach Anspruch 1, wobei BHT mit 2 mg pro kg der Zusammensetzung bis 140 mg pro kg der Zusammensetzung, oder 6 mg pro kg der Zusammensetzung bis 80 mg pro kg der Zusammensetzung, oder 10 mg pro kg der Zusammensetzung bis 60 mg pro kg der Zusammensetzung vorliegt.

4. Tiernahrungszusammensetzung nach einem der vorstehenden Ansprüche, wobei BHA und/oder BHT allein oder in Kombination miteinander mit 2 mg pro kg der Zusammensetzung bis 140 mg pro kg der Zusammensetzung, oder 3 mg pro kg der Zusammensetzung bis 120 mg pro kg der Zusammensetzung, oder 4 mg pro kg der Zusammensetzung bis 100 mg pro kg der Zusammensetzung, oder 5 mg pro kg der Zusammensetzung bis 90 mg pro kg der Zusammensetzung, oder 6 mg pro kg der Zusammensetzung bis 80 mg pro kg der Zusammensetzung, oder 10 mg pro kg der Zusammensetzung bis etwa 30 mg pro kg der Zusammensetzung vorliegen.

5. Tiernahrungszusammensetzung nach einem der vorstehenden Ansprüche, umfassend BHA und BHT.

6. Tiernahrungszusammensetzung nach Anspruch 5, wobei das Verhältnis von BHA zu BHT etwa 1:1 beträgt.

7. Tiernahrungszusammensetzung nach einem der vorstehenden Ansprüche, wobei der Glucose-Antimetabolit in der Tiernahrungszusammensetzung mit weniger als etwa 5 Gew.-% der Zusammensetzung vorliegt.

8. Tiernahrungszusammensetzung nach einem der vorstehenden Ansprüche, wobei der Glucose-Antimetabolit Mannoheptulose umfasst.

9. Tiernahrungszusammensetzung nach einem der vorstehenden Ansprüche, wobei die Tiernahrungszusammensetzung eine Zusammensetzung für Hunde oder Katzen ist.

10. Tiernahrungszusammensetzung nach einem der vorstehenden Ansprüche, wobei die Tiernahrungszusammensetzung ferner zu 10 % bis 90 % Rohprotein oder von 20 % bis 50 % Rohprotein, oder von 20 % bis 40 % Rohprotein oder von 20 % bis 35 % Rohprotein, bezogen auf das Gewicht der Zusammensetzung, in der Trockenmasse umfasst.

11. Tiernahrungszusammensetzung nach einem der vorstehenden Ansprüche, wobei die Tiernahrungszusammensetzung ferner zu 5 % bis 40 % Fett, bezogen auf das Gewicht der Zusammensetzung, und von 10 % bis 35 % Fett, bezogen auf das Gewicht der Zusammensetzung, in der Trockenmasse umfasst.

12. Tiernahrungszusammensetzung nach einem der vorstehenden Ansprüche, wobei die Tiernahrungszusammensetzung ferner zu 35 % bis 50 %, bezogen auf das Gewicht der Zusammensetzung, eine Kohlenhydratquelle, oder zu 35 % bis 45 %, bezogen auf das Gewicht der Zusammensetzung, eine Kohlenhydratquelle, oder zu 40 % bis 50 %, bezogen auf das Gewicht der Zusammensetzung, eine Kohlenhydratquelle umfasst.

13. Tiernahrungszusammensetzung nach Anspruch 12, wobei die Kohlenhydratquelle Reis, Mais, Milo, Sorghum, Gerste, Weizen und/oder Kombinationen davon umfasst.

14. Tiernahrungszusammensetzung nach einem der vorstehenden Ansprüche, ferner umfassend getrocknete Molke, Milch-Nebenprodukte, Rübenschnitzel, Zellulosefaser, Fischöl, Flachs, Vitamine, Mineralien, Aromen, Antioxidantien und/oder Taurin.

15. Tiernahrungszusammensetzung nach einem der vorstehenden Ansprüche zur Verwendung in einem Verfahren zur therapeutischen Behandlung des Tierkörpers.


**Revendications**

1. Composition alimentaire pour animaux de compagnie comprenant un antimétabolite du glucose, et du BHA et/ou BHT ;
   dans laquelle l'antimétabolite du glucose est choisi dans le groupe constitué de 2-désoxy-D-glucose, 5-thio-D-glucose, 3-O-méthylglucose, 1,5-anhydro-D-glucitol, 2,5-anhydro-D-glucitol, 2,5-anhydro-D-mannitol, mannoheptulose, et des mélanges et combinaisons de ceux-ci ; et
   dans laquelle la composition alimentaire pour animaux de compagnie est nutritionnellement équilibrée.

2. Composition alimentaire pour animaux de compagnie selon la revendication 1, dans laquelle le BHA est présent à raison de 2 mg par kg de composition à 140 mg par kg de composition, ou de 6 mg par kg de composition à 80 mg par kg de composition, ou de 10 mg par kg de composition à 60 mg par kg de composition.

3. Composition alimentaire pour animaux de compagnie selon la revendication 1, dans laquelle le BHT est présent à raison de 2 mg par kg de composition à 140 mg par kg de composition, ou de 6 mg par kg de composition à 80 mg par kg de composition, ou de 10 mg par kg de composition à 60 mg par kg de composition.

4. Composition alimentaire pour animaux de compagnie selon l'une quelconque des revendications précédentes, dans laquelle le BHA et/ou le BHT sont présents, seuls ou en combinaison l'un avec l'autre, à raison de 2 mg par kg de composition à 140 mg par kg de composition, ou de 3 mg par kg de composition à 120 mg par kg de composition, ou de 4 mg par kg de composition à 100 mg par kg de composition, ou de 5 mg par kg de composition à 90 mg par kg de composition, ou de 6 mg par kg de composition à 80 mg par kg de composition, ou de 10 mg par kg de composition à environ 30 mg par kg de composition.

5. Composition alimentaire pour animaux de compagnie selon l'une quelconque des revendications précédentes comprenant du BHA et du BHT.

6. Composition alimentaire pour animaux de compagnie selon la revendication 5, dans laquelle le rapport du BHA au BHT est d'environ 1:1.

7. Composition alimentaire pour animaux de compagnie selon l'une quelconque des revendications précédentes, dans laquelle l'antimétabolite du glucose est présent dans la composition alimentaire pour animaux de compagnie à raison de moins d'environ 5 % en poids de la composition.

8. Composition alimentaire pour animaux de compagnie selon l'une quelconque des revendications précédentes, dans laquelle l'antimétabolite du glucose comprend du mannoheptulose.

9. Composition alimentaire pour animaux de compagnie selon l'une quelconque des revendications précédentes, dans laquelle la composition alimentaire pour animaux de compagnie est une composition pour chiens ou chats.

10. Composition alimentaire pour animaux de compagnie selon l'une quelconque des revendications précédentes, dans laquelle la composition alimentaire pour animaux de compagnie comprend en outre, sur une base de matière sèche, de 10 % à 90 % de protéine brute, ou de 20 % à 50 % de protéine brute, ou de 20 % à 40 % de protéine brute, ou de 20 % à 35 % de protéine brute, en poids de la composition.

11. Composition alimentaire pour animaux de compagnie selon l'une quelconque des revendications précédentes, dans laquelle la composition alimentaire pour animaux de compagnie comprend en outre, sur une base de matière sèche, de 5 % à 40 % de matière grasse, en poids de la composition, ou de 10 % à 35 % de matière grasse, en poids de la composition.

12. Composition alimentaire pour animaux de compagnie selon l'une quelconque des revendications précédentes, dans laquelle la composition alimentaire pour animaux de compagnie comprend en outre de 35 % à 50 %, en poids de la composition, d'une source de glucides, ou de 35 % à 45 %, en poids de la composition, d'une source de glucides, ou de 40 % à 50 %, en poids de la composition, d'une source de glucides.

13. Composition alimentaire pour animaux de compagnie selon la revendication 12, dans laquelle la source de glucides comprend du riz, du maïs, du sorgho milo, du sorgho, de l'orge, du blé et/ou des combinaisons de ceux-ci.

14. Composition alimentaire pour animaux de compagnie selon l'une quelconque des revendications précédentes, comprenant en outre du lactosérum déshydraté, des sous-produits laitiers, de la pulpe de betterave, de la fibre de cellulose, de l'huile de poisson, du lin, des vitamines, des minéraux, des arômes, des antioxydants, et/ou de la taurine.

15. Composition alimentaire pour animaux de compagnie selon l'une quelconque des revendications précédentes pour utilisation dans une méthode de traitement du corps de l'animal par thérapie.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2663198
- WO 2008093302 A2
- US 20050158294 A

**Non-patent literature cited in the description**

- **WEINDRUCH ; WALFORD.** The Retardation of Aging and Disease by Dietary Restriction. 1988
- **YU.** Modulation of Aging Processes by Dietary Restriction. CRC Press, 1994
- **FISHBEIN.** Biological Effects of Dietary Restriction. Springer, 1991
- **MCKAY et al.** The Effect of Retarded Growth Upon the Length of Lifespan and Upon Ultimate Body Size. *J. Nutr.,* 1935, vol. 10, 63-79
- **MASORO, E.J.** Overview of Caloric Restriction and Ageing. *Mech. Aging Dev.,* 2005, vol. 126, 913-922
- **KALANT et al.** Effect of Diet Restriction on Glucose Metabolism and Insulin Responsiveness and Aging Rats. *Mech. Aging Dev.,* 1988, vol. 46, 89-104
- **BALKAU ; ESCHWEGE.** *Diabetes Obes. Metab.,* 1999, vol. 1 (1), 23-31
- **MASORO et al.** *J. Gerontol. Biol. Sci.,* 1992, vol. 47, B202-B208
- **KOIZUMI et al.** *J. Nutr.,* 1987, vol. 117, 361-367
- **LANE et al.** *Proc. Nat. Acad. Sci.,* 1996, vol. 93, 4154-4164
- **REZEK et al.** *J. Nutr.,* 1972, vol. 106, 143-157
- **ROTH et al.** *Ann. NY Acad. Sci.,* 2001, vol. 928, 305-315
- **GRAJALES-LAGUNES et al.** *Drying Technology,* 1999, vol. 17 (1 &2), 317-326
- **BOTTERWECK et al.** Intake of Butylated Hydroxyanisole and Butylated Hydroxytoluene and Stomach Cancer Risk: Results from Analyses in the Netherlands Cohort Study. *Food and Chemical Toxicology,* 2000, vol. 38, 599-605
- **SHAW, P.E. ; WILSON, C.W. ; KNIGHT, R.J.** *J. Agric. Food Chem.,* 1980, vol. 28, 379-382